# EUROPEAN PATENT APPLICATION

(11) **EP 2 258 370 A1**
(43) Date of publication of application: **08.12.2010**
(21) Application number: 09162020.3
(22) Date of filing: 05.06.2009
(51) Int. Cl.: A61K 31/506, A61P 35/00

(54) **Treatment of malignant diseases**

(71) Applicant: Medizinische Universität Wien, 1090 Wien (AT)
(72) Inventor: Seyedhossein, Aharinejad, 1130, Vienna (AT)
(74) Representative: Sonn & Partner Patentanwälte

(57) **Abstract**

The invention discloses the use of a 1H-indol-3-yl-compound with the formula (I) 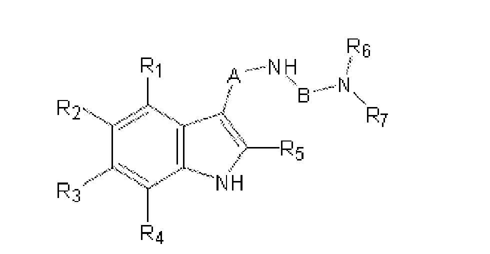 and any of its stereoisomeric forms or a mixture thereof wherein
R₁, R₃, R₄ and R₅ are H;
R₇ is H or OH;
A is CH₂-CH₂;
B is 6-CH₃-pyrimidine;
R₆ is H; and
R₇ is CHR₈ (CH₂) ₃NR₉R₁₀; wherein R₃ is CH₃; R₉ and R₁₀ are C₂H₅; or a salt or solvate of a compound of formula (I) for the treatment of malignant diseases.

## Description

The present invention relates to the use of inhibitors of Ras GTPases for the treatment of malignant diseases.

Rho family GTPases are important intracellular signaling proteins that control diverse cellular functions related to cancer development, including actin cytoskeleton organization, transcription regulation, cell cycle progression, apoptosis, vesicle trafficking, and cell-to-cell and cell-to-extracellular matrix adhesions. They have emerged as potentially useful targets for tumourigenesis. In particular, several lines of evidence indicate that the Rho family member, Rac, may play critical roles in several aspects of tumourigenesis and cancer progression. First, constitutively active Rac1 could accelerate cell growth and transform NIH 3T3 cells, whereas dominant negative Rac inhibited cell growth. Second, Rac1 was found to be an important component of Ras-induced transformation, and the Rac-specific GEF, Tiam1, is involved in Ras-mediated skin cancer progression in mice. Third, loss of function in PTEN, p19Arf, or p53 tumour suppressor leads to elevation of Rac activity, resulting in increased migration and proliferation in PTEN^{-/-}, p19Arf^{-/-}, or p53^{-/-} cells. Fourth, hyperactive Rac1 and Rac3 were associated with the increased proliferation of several breast cancer cell lines. In addition, a point mutation of Tiam1 has been identified in human tumours, and this mutant was shown to transform NIH 3T3 cells. Unlike Ras, however, constitutively active mutation of Rac has not been found in human cancer. It is possible that up-regulation or overexpression, rather than GTPase-defective mutation, of Rac is associated with tumouri-genic properties. Therefore, the signaling step of Rac1 activation by GEF could be a targeting site of the signaling chains involving Rac. Based on the structural information provided by the Rac1-Tiam1 complex and by taking the computer-simulated virtual screening approach, the small chemical compound of the National Cancer Institute chemical database, NSC23766 (N6-(2-((4-(diethylamino)-1- methylbutyl) amino)-6-methyl-4-pyrimidinyl)-2-methyl-4, 6-quinolinediamine), was identified as a Rac-specific inhibitor (Gao et al., PNAS 101 (2004), 7618-7623; WO 2005/051392 A1). It was shown that this compound was able to discriminate Rac1 from Cdc42 and RhoA and specifically inhibited Rac1 activation by its GEF in vitro and in vivo. This compound was able to inhibit proliferation, anchorage independent growth, and invasion of the Rac-hyperactive prostate cancer cells (in a human cancer cell line, PC-3) by down-regulating the endogenous Rac1 activity. As a first generation inhibitor for Rac, NSC23766 is capable of specifically interfering with Rac1 but not with Cdc42 or RhoA activation by their respective GEFs, as demonstrated by the in vitro binding and exchange assays and the in vivo Rho GTPase effector domain pull-down assays.

It is an object of the present invention to provide further substances treating tumours identified by their Rac inhibiting property. These substances should be small molecules with molecular weights not exceeding 1000 (or even 500) Da.

Therefore, the present invention relates to the use of a compound of the formula (I) 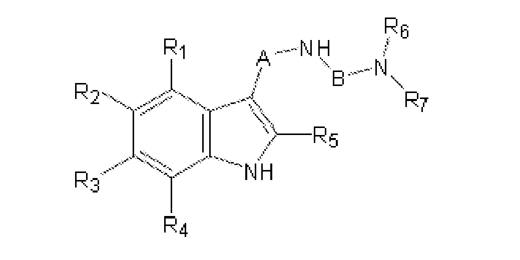 and any of its stereoisomeric forms or a mixture thereof wherein
R₁, R₃, R₄ and R₅ are H;
R₂ is H or OH;
A is CH₂-CH₂;
B is 6-CH₃-pyrimidine;
R₆ is H; and
R₇ is CHR₈(CH₂)₃NR₉R₁₀; wherein R₈ is CH₃; R₉ and R₁₀ are C₂H₅;
or a salt or solvate of a compound of formula (I) for the treatment of malignant diseases.

These compounds N2-(4-Diethylamino-1-methyl-butyl)-N4-[2-(1H-indol-3-yl)-ethyl]-6-methyl-pyrimidine-2,4-diamine and 3-{2-[2-(4-Diethylamino-1-methyl-butylamino)-6-methyl-pyrimidin-4-ylamino]-ethyl}-1H-indol-5-ol which are hereinafter referred to as "the compounds according to the present invention" have been identified in the course of the present invention as being potent and specific Rac1 inhibitors. The compounds according to the present invention are efficient in a broad range of malignancies, since the Rac system plays a role in solid malignant tumours as well as in hematopoietic malignancies. Accordingly, the compounds of the present invention may preferably be used for the treatment of carcinomas (including breast, colon, rectum, esophagus, stomach, liver, lung, renal (especially renal cell tumour and adrenal tumour), thyroid, ovary, uterus, vagina, pancreas, bladder, prostate, skin, and head and neck squamous cell carcinoma (HNSCC), as well as oral squamous cell carcinoma (OSCC)), hematopoietic malignancies of lymphoid lineage (including anaplastic large cell lymphomas (ALCLs), p210-BCR-ABL-dependent leukemia and hairy cell leukemia), hematopoietic malignancies of myeloid lineage (including chronic myelogenous leukemias (CML)), malignant tumours of mesenchymal origin (including rhabdomyosarcoma), and malignant tumours of neuronal origin, especially glioblastoma.

The route of administration can be chosen and optimised by the physician based on the tumour type, the physical status of the patient (and the tumour stage) and the respective compound. The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels or aqueous or oily solutions or suspensions) for administration by inhalation (for example as a finely divided powder or a liquid aerosol), for administration by insufflation (for example as a finely divided powder) or for parenteral administration (for example as a sterile aqueous or oily solution for intravenous; subcutaneous, intramuscular application) or as a suppository for rectal application, as well as use of infusion techniques. Intratumoural injections can be used for treatment of cancers as well.

One embodiment provides for a method for reducing cancer cell proliferation by administering in a subject having cancer an effective amount of a compound according to the present invention.

Another embodiment provides for the use of an effective amount of an active compound according to the present invention for the preparation of pharmaceutical composition for the treatment of a disease associated with abnormal cell proliferation.

Because of their cell proliferation inhibitory activity, the compounds according to the present invention are suitable for treating a variety of malignant diseases in a variety of conditions. Accordingly, the compounds can be used at very early stages of a disease, or after significant progression, including metastasis. The term "treatment" or "treating" designates in particular a reduction of the burden in a patient (whom is hereinafter always understood as human patient, except explicitly indicated otherwise; this holds also true for the using of the words "body" or "cell", which - unless explicitly stated otherwise - refer to human cells or the human body), such as a reduction in cell proliferation rate, a destruction of diseased proliferative cells, a reduction of tumour mass or tumour size, a delaying of tumour progression, as well as a complete tumour suppression.

The compounds of the preferred embodiments are particularly suited for the treatment of cancers, such as solid malignant tumours or malignancies of the hematopoietic system. Specific examples include leukemia, prostate cancer, ovarian cancer, uterine cancer, vaginal cancer, pancreas cancer, lung cancer, breast cancer, liver cancer, head and neck cancer, colon cancer, rectal cancer, esophagus cancer, stomach cancer, skin cancer, bladder cancer and non-Hodgkin's lymphoma cancer. The most preferred embodiments include the treatment of colon cancer and breast cancer.

The active compounds of the present invention can be administered through various routes, typically by injection, such as local or systemic injection(s). Intratumoural injections can be also used for treating existing cancers. However, other administration routes can be used as well, such as intramuscular, intravenous, intradermic, subcutaneous, etc. Furthermore, repeated injections can be performed, if needed, although it is believed that limited injections will be needed in view of the efficacy of the compounds.

It is contemplated that such target cells can be located within an animal or a patient, in which case a safe and effective amount of the complex, in pharmacologically acceptable form, would be administered. Generally speaking, it is contemplated that useful pharmaceutical compositions of the preferred embodiments will include the selected active compound derivative in a convenient amount, e. g., from about 0.001% to about 10%

(w/w) that is diluted in a pharmacologically or physiologically acceptable carrier, such as, for example, phosphate buffered saline. The route of administration and ultimate amount of material that is administered to the patient or animal under such circumstances will depend upon the intended application and will be apparent to those of skill in the art in light of the examples which follow.

Any composition chosen should be of low or non-toxicity to the cell or to the body in general. Toxicity for any given compound can vary with the concentration of compound used. It is also beneficial if the compound chosen is metabolized or eliminated by the body and if this metabolism or elimination is done in a manner that will not be harmfully toxic. In any way, the compounds of the present invention are preferably administered such that a therapeutically effective concentration of the compound is in contact with the affected cells of the body.

The dose administered, particularly to a patient, in the context of the preferred embodiments is preferably sufficient to effect a therapeutic response in the patient over a reasonable period of time. The dose will be determined by the strength of the particular compound employed and the condition of the patient, as well as the body weight of the patient to be treated. The existence, nature, and extent of any adverse side effects that might accompany the administration of a particular compound also will determine the dose and the particular route of administration employed with a particular patient and a particular cancer type. In general, the compounds of the preferred embodiments are therapeutically effective at low doses. The generally preferred dose range is from about 0.0001 to about 10 mM. Preferably, the effective dose range is from about 0.001, 0.005, 0.01, 0.05, 0.06, 0.07, 0.08, or 0.09 mM, to about 0.1, 0.2, 0.3, 0.4, 0.5, 0.6, 0.7, 0.8, 0.9, or 1 mM.

The compound can be administered in a pharmaceutically acceptable carrier. Pharmaceutically acceptable carriers are well-known to those who are skilled in the art. The choice of carrier will be determined in part by the particular compound, as well as by the particular method used to administer the composition. Accordingly, there is a wide variety of suitable formulations of the pharmaceutical composition of the preferred embodiments.

The compounds can be administered orally, topically, parenterally, by inhalation or spray, vaginally, rectally or sublingually in dosage unit formulations. The term "administration by injection" includes but is not limited to: intravenous, intraarticular, intramuscular, subcutaneous and parenteral injections, as well as use of infusion techniques. For example, osmotic minipumps or different types of depots may be applied which can be implanted to deliver a daily, two-day, weekly, biweekly, etc. dose in a controlled manner.

Dermal administration can include topical application or transdermal administration. One or more compounds can be present in association with one or more non-toxic pharmaceutically acceptable carriers and if desired other active ingredients.

Compositions intended for oral use can be prepared according to any suitable method known to the art for the manufacture of pharmaceutical compositions.

Such compositions can contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients that are suitable for the manufacture of tablets. These excipients can be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets can be uncoated or they can be coated by known techniques to delay disintegration and adsorption in the gastrointestinal tract and thereby provide a sustained action over a longer period. For example, a time delay material such as glyceryl monostearate or glyceryl distearate can be employed. These compounds can also be prepared in solid, rapidly released form.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions can also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, can also be present.

The compounds according to the present invention can also be in the form of non-aqueous liquid formulations, e. g., oily suspensions which can be formulated by suspending the active ingredients in a vegetable oil, for example arachis oil, olive oil, sesame oil or peanut oil, or in a mineral oil such as liquid paraffin. The oily suspensions can contain a thickening agent, for example beeswax, hard paraffin or cetyl alcohol. Sweetening agents such as those set forth above, and flavoring agents can be added to provide palatable oral preparations. These compositions can be preserved by the addition of an anti-oxidant such as ascorbic acid.

Compounds according to the present invention can also be administrated transdermally using methods known to those skilled in the art. For example, a solution or suspension of an active agent in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of an active agent can be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents can also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to about 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations can also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates can also be used as matrix components. Additional additives, such as viscous resins or oils can be added to increase the viscosity of the matrix.

Pharmaceutical compositions according to the present invention can also be in the form of oil-in-water emulsions. The oil phase can be a vegetable oil, for example olive oil or arachis oil, or a mineral oil, for example, liquid paraffin or mixtures of these. Suitable emulsifying agents can be naturally-occurring gums, for example, gum acacia or gum tragacanth, naturally-occurring phosphatides, for example, soy bean, lecithin, and esters or partial esters derived from fatty acids and hexitol anhydrides, for example, sorbitan monooleate, and condensation products of the said partial esters with ethylene oxide, for example, polyoxyethylene sorbitan monooleate. The emulsions can also contain sweetening and flavoring agents. Syrups and elixirs can be formulated with sweetening agents, for example glycerol, propylene glycol, sorbitol or sucrose. Such formulations can also contain a demulcent, a preservative and flavoring and coloring agents.

The compounds of the present invention can also be administered in the form of suppositories for rectal or vaginal administration of the drug. These compositions can be prepared by mixing the drug with a suitable nonirritating excipient which is solid at ordinary temperatures but liquid at the rectal temperature or vaginal temperature and will therefore melt in the rectum or vagina to release the drug. Such materials include cocoa butter and polyethylene glycols.

For all regimens of use disclosed herein for active agent, the daily dosage regimen will preferably be from about 0.0001 mg to about 100 mg, especially from 0.005 mg to 50 mg/Kg of total body weight. Preferably, the daily oral dosage regimen will be from about 0.001 mg to about 20 mg/Kg of total body weight. The daily dosage for administration by injection, including intravenous, intramuscular, subcutaneous and parenteral injections, and use of infusion techniques will preferably be from 0.0001 to 2 mg/Kg of total body weight.

Preferably, the daily dosage for administration by injection, including intravenous, intramuscular, subcutaneous and parenteral injections, and use of infusion techniques will preferably be from about 0.0001, 0.0005, 0.001, 0.005, 0.01, 0.02, 0.03, 0.04, or 0.05 to about 0.1, 0.5, 1, 1.1, 1.2, 1.3, 1.4, 1.5, 1.6, 1.7, 1.8, 1.9, 2, or 4 mg/Kg of total body weight. The daily vaginal dosage regime will preferably be from 0.001 to 20 mg/Kg of total body weight. The daily topical dosage regimen will preferably be from 0.01 to 40 mg /Kg administered between one to four times daily. The concentration for vaginal dosage and topical dosage will preferably be that required to maintain a daily dosage from 0.01 to 40 mg/Kg. Preferably, the daily oral dosage regimen will be from about 0.001, 0.005, 0.01, 0.05, 0.1, 0.2, 0.3, 0.4, or 0.5 to about 1, 5, 10, 11, 12, 13, 14, 15, 16, 17, 18, 19, or 20 mg/Kg of total body weight. The daily inhalation dosage regimen will preferably be from 0.001 to 20 mg/Kg of total body weight. Preferably, the daily inhalation dosage regimen will be from about 0.001, 0.005, 0.01, 0.05, 0.1, 0.2, 0.3, 0.4, or 0.5 to about 1, 5, 10, 11, 12, 13, 14, 15, 16, 17, 18, 19, or 20 mg/Kg of total body weight.

It will be appreciated by those skilled in the art that the particular method of administration will depend on a variety of factors, all of which are considered routinely when administering therapeutics, especially tumour therapeutics. It will also be understood, however, that the specific dose level for any given patient will depend upon a variety of factors, including, the activity of the specific compound employed, the age of the patient, the body weight of the patient, the general physical condition of the patient, the gender of the patient, the diet of the patient, time of administration, route of administration, rate of excretion, drug combinations, and the severity and stage of the disease undergoing therapy. It will be further appreciated by one skilled in the art that the optimal course of treatment, i. e., the mode of treatment and the daily number of doses of an active agent or a pharmaceutically acceptable salt thereof given for a defined number of days, can be ascertained by those skilled in the art using conventional treatment tests.

The modulation of oncogenic transforming activity by an active agent according to the present invention can be applied in practice by various procedures.

For example, the present invention relates to a method of treating cancer comprising administering, to a subject in need of treatment, an amount of a compound according to the present invention effective to treat the disease. Treating the disease can mean, delaying its onset, delaying the progression of the disease, improving or delaying clinical and pathological signs of disease.

The present invention provides an improved method for treatment of malignant diseases comprising administration of a pharmaceutically effective quantity of the compounds according to the present invention or its pharmaceutically acceptable salts or esters, active agent analogs or their pharmaceutically acceptable salts or esters, or a combination thereof.

The compositions and preparations described preferably contain at least 0.01% (weight percentage), preferably at least 0.1%, of a compound according to the present invention. The percentage of the compositions and preparations can, of course, be varied, and can contain between about 0.2% and 60%, preferably between 0.3% to 10%, especially 0.5% to 5%, of the weight of the amount administered. The amount of active compounds in such pharmaceutically useful compositions and preparations is such that a suitable dosage will be obtained.

The compounds according to the present invention may also be administered in the form of salts. Accordingly, any reference to a compound of the present invention as used herein is understood to include reference to salts thereof, unless otherwise indicated. The term "salt(s)", as employed herein, denotes acidic and/or basic salts formed with inorganic and/or organic acids and bases. In addition, since the active agent according to the present invention contains a pyridine and indol ring as a basic moiety, salts involving these basic moieties in salt formation are specifically preferred. Pharmaceutically acceptable (i. e., non-toxic, physiologically acceptable) salts are preferred, although other salts are also useful, e. g., in isolation or purification steps, which can be employed during preparation. Salts of the compounds of the active agent can be formed, for example, by reacting a compound of the present invention with an amount of acid or base, such as an equivalent amount, in a medium such as one in which the salt precipitates or in an aqueous medium followed by lyophilization.

Since the active agent according to the present invention contains basic moieties, such as the pyridine or indol ring, salts can easily be formed with a variety of organic and inorganic acids. Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecylsulfates, ethane-sulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, 2-hydroxyethanesulfonates, lactates, maleates (formed with maleic acid), methanesulfonates (formed with methanesulfonic acid), 2-naphthalenesulfonates, nicotinates, nitrates, oxalates, pectinates, persulfates, 3- phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

Prodrugs and solvates of the compounds of the present invention are also contemplated herein. The term "prodrug", as employed herein, denotes a compound which, upon administration to a subject, undergoes chemical conversion by metabolic or chemical processes to yield a compound of the present invention, and/or a salt and/or solvate thereof. Solvates of the active agent are preferably hydrates.

All stereoisomers of the present compounds, such as those, for example, which can exist due to asymmetric carbons on any of the substituents, including enantiomeric forms (which can exist even in the absence of asymmetric carbons) and diastereomeric forms, are contemplated and within the scope of the preferred embodiments. Individual stereoisomers of the compounds of the preferred embodiments can, for example, be substantially free of other isomers, or can be admixed, for example, as racemates or with all other or other selected, stereoisomers. The chiral centers of the preferred embodiments can have the S or R configuration as defined by the IUPAC 1974 Recommendations.

When the compounds according to the present invention are in the forms of salts, they are preferably pharmaceutically acceptable salts. Such salts include pharmaceutically acceptable acid addition salts, pharmaceutically acceptable base addition salts, pharmaceutically acceptable metal salts, ammonium and alkylated ammonium salts.

Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

Mixture of solvents can be used. Organic bases like lysine, arginine, diethanolamine, choline, guandine and their derivatives etc. can also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, sulfonic acid, acetic acid, citric acid, maleic acid, salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane, etc. Mixtures of solvents can also be used.

The compounds according to the present invention can be formulated in various forms, including solid and liquid forms, such as tablets, gel, syrup, powder, aerosol, etc.

The compositions to be administered according to the present invention can contain physiologically acceptable diluents, fillers, lubricants, excipients, solvents, binders, stabilizers, and the like. Diluents that can be used in the compositions include but are not limited to dicalcium phosphate, calcium sulphate, lactose, cellulose, kaolin, mannitol, sodium chloride, dry starch, powdered sugar and - for prolonged release tablets - hydroxy propyl methyl cellulose (HPMC). The binders that can be used in the compositions include but are not limited to starch, gelatin and fillers such as sucrose, glucose, dextrose and lactose.

Natural and synthetic gums that can be used in the compositions include but are not limited to sodium alginate, ghatti gum, carboxymethyl cellulose, methyl cellulose, polyvinyl pyrrolidone and veegum. Excipients that can be used in the compositions include but are not limited to microcrystalline cellulose, calcium sulfate, calcium phosphate, starch, magnesium stearate, lactose, and sucrose. Stabilizers that can be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.

Solvents that can be used include but are not limited to Ringers solution, water, distilled water, dimethyl sulfoxide to 50% in water, propylene glycol (neat or in water), phosphate buffered saline, balanced salt solution, glycol and other conventional fluids.

The compounds of the present invention can be used in a substantially similar manner to other known anti-tumour agents for treating (both chemopreventively and therapeutically) various malignant diseases. For the compounds of the present invention, the anti-tumour dose to be administered, whether a single dose, multiple doses, or a daily dose, will of course vary with the particular compound employed because of the varying potency of the compound, the chosen route of administration, the body weight and body surface of the recipient, the type of tumour, and the patient's physical condition and disease stage. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. A skilled physician in the art of cancer treatment will be able to ascertain, without undue experimentation, appropriate protocols for the effective administration of the compounds of the preferred embodiments, such as by referring to the earlier published studies on compounds found to have anti-tumour properties.

The present invention is further disclosed by the following examples and the drawing figures, yet without being restricted thereto.

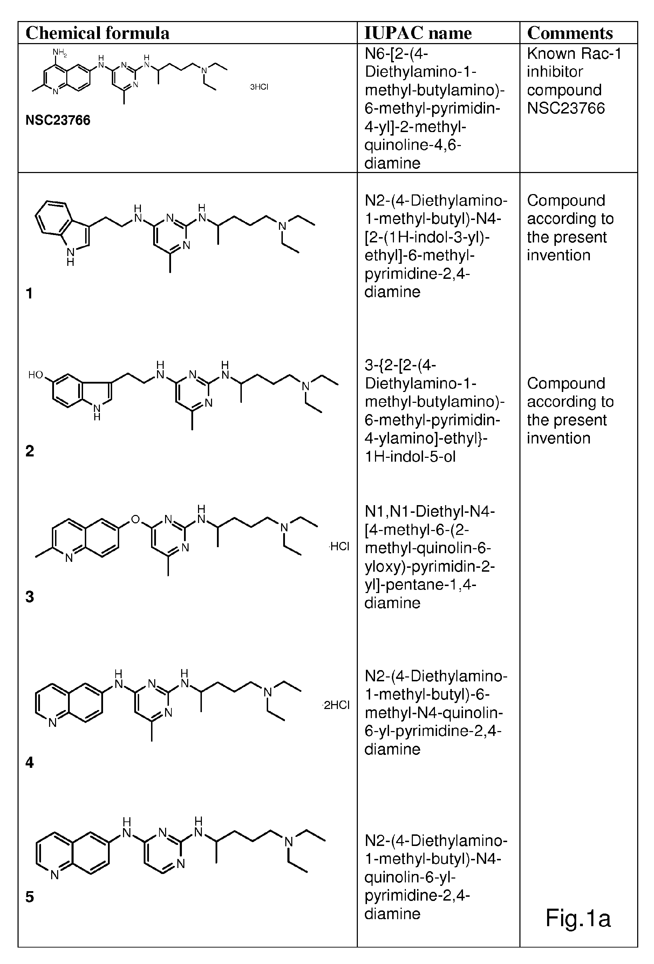

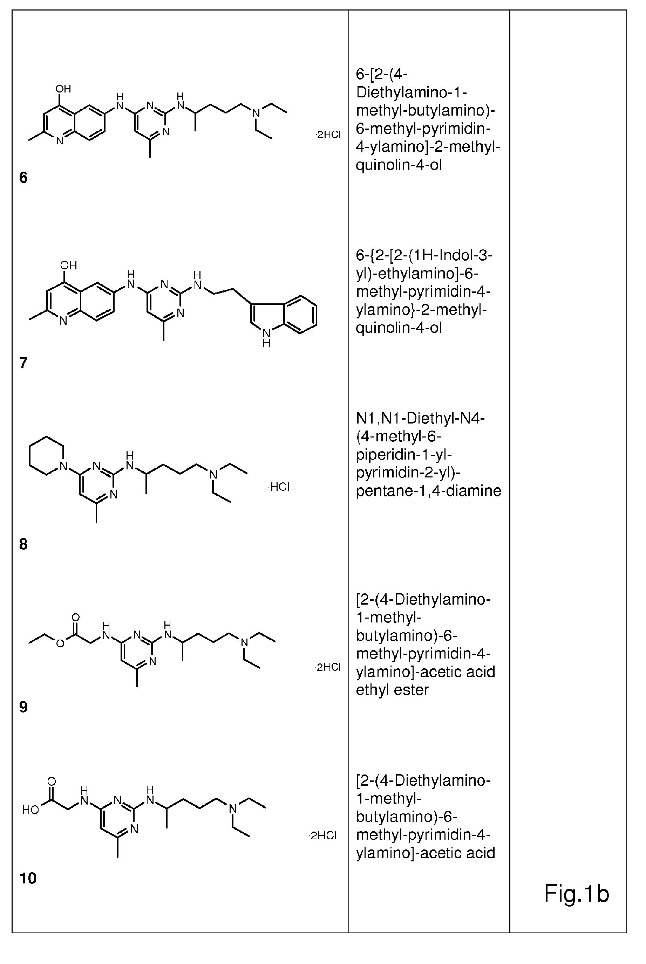

Figs.1 a and b show the chemical formulas of the compounds according to the present invention and the formula compounds used for comparative experiments;
Figs.2a to f show the results of the proliferation assays;
Fig.3a shows tumour volumes in mice bearing human breast cancer xenografts; days post injectionem (d.p.i.); Fig.3b shows mean breast cancer xenograft masses in control mice and mice treated with 25, 50 and 100 µg of Rac-1 inhibitor (*: significantly different from control (Co), P<0.05); Fig.3c shows body weights of mice bearing human breast cancer xenografts (*: significantly different from control (Co), P<0.05); Fig.3d shows representative images of mice bearing breast cancer xenografts treated with 25, 50 and 100 µg of Rac-1 inhibitor (*: significantly different from control (Co), P<0.05);
Fig.4 shows the mean colon cancer xenograft masses in control mice and mice treated with 100 µg/day of differrent Rac-1 inhibitors;
Fig.5 shows body weights of mice bearing human colon cancer xenografts on day 22.

### EXAMPLES

### Example 1:

### Synthesis of N2-(4-Diethylamino-1-methyl-butyl)-N4-[2-(1H-indol-3-yl)-ethyl]-6-methyl-pyrimidine-2,4-diamine and 3-{2-[2-(4-Diethylamino-1-methyl-butylamino)-6-methyl-pyrimidin-4-ylamino]-ethyl}-1H-indol-5-ol

### N2-(4-Diethylamino-1-methyl-butyl)-N4-[2-(1H-indol-3-yl)-ethyl]-6-methyl-pyrimidine-2,4-diamine

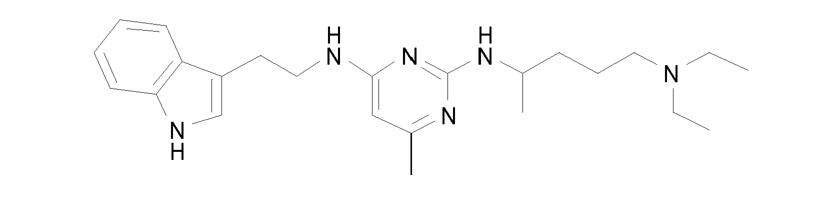

and 3-{2-[2-(4-Diethylamino-1-methyl-butylamino)-6-methyl-pyrimidin-4-ylamino]-ethyl}-1H-indol-5-ol 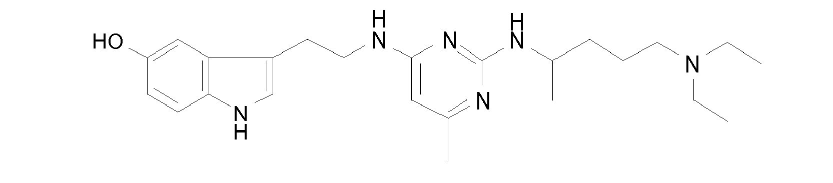 are hereinafter also referred to as compounds "1" and "2", respectively, can be produced by well available methods and syntheses. Compounds 1 and 2 used in the present examples have been produced according to the following method:

### Compound 1:

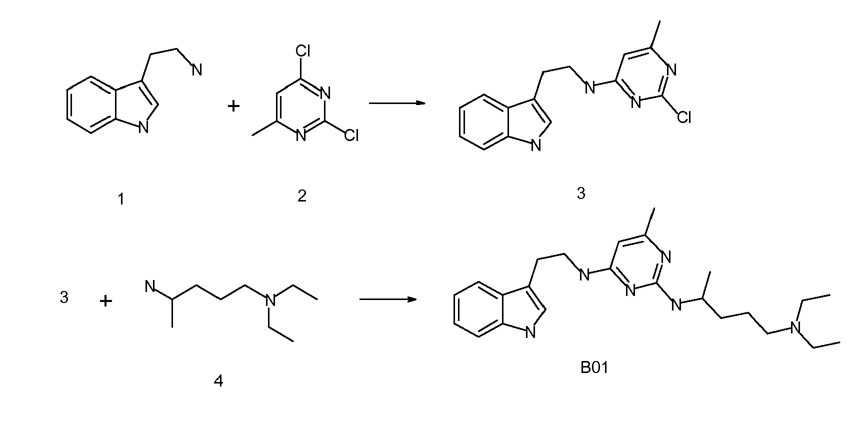

An equimolar amount of compounds 1 and 2 were reacted in MeCN using Et₃N as a base. Compound 3 was liberated by column chromatography from its unwanted isomer and reacted with amine 4, neat, under microwave conditions. Purification by column chromatography gave pure Compound 1 ("B01" in the above scheme).

### Compound 2:

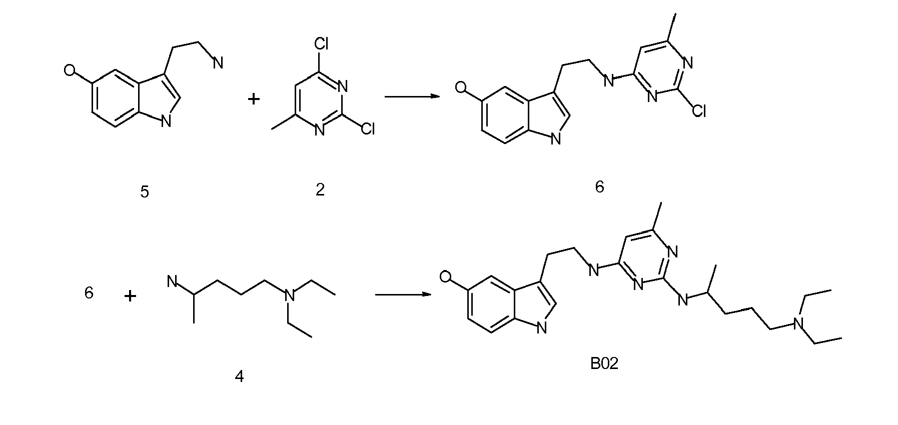

An equimolar amount of compounds 5 and 2 were reacted in MeCN using Et₃N as a base. Compound 6 was liberated by column chromatography from its unwanted isomer and reacted with amine 4, neat, under microwave conditions. Purification by column chromatography gave pure Compound 2 ("B02" in the above scheme).

### Example 2:

### Proliferation assay for testing Rac-1 inhibitor compounds

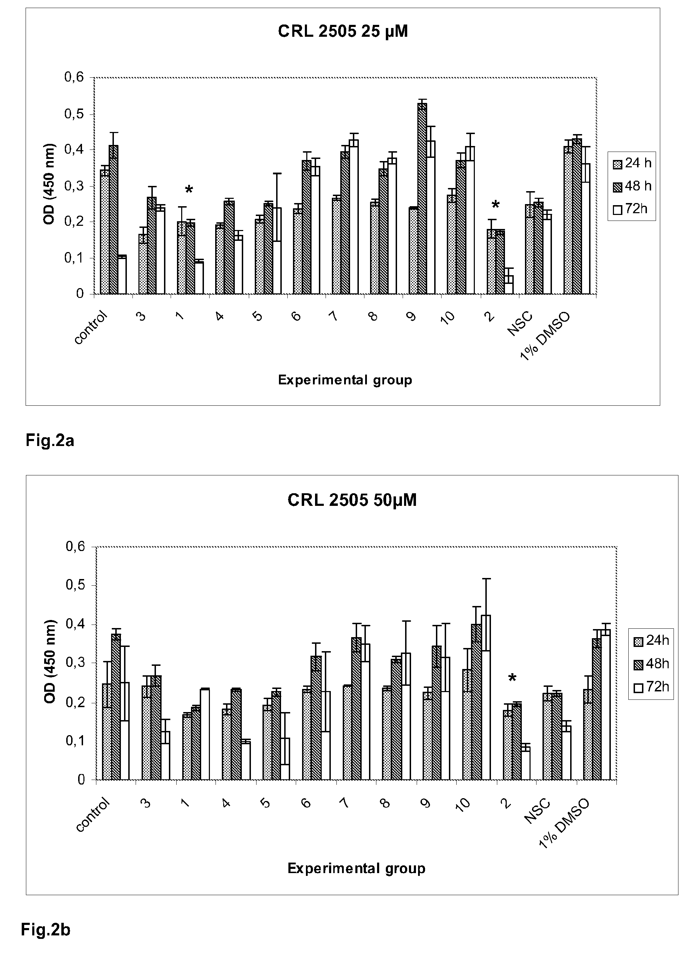

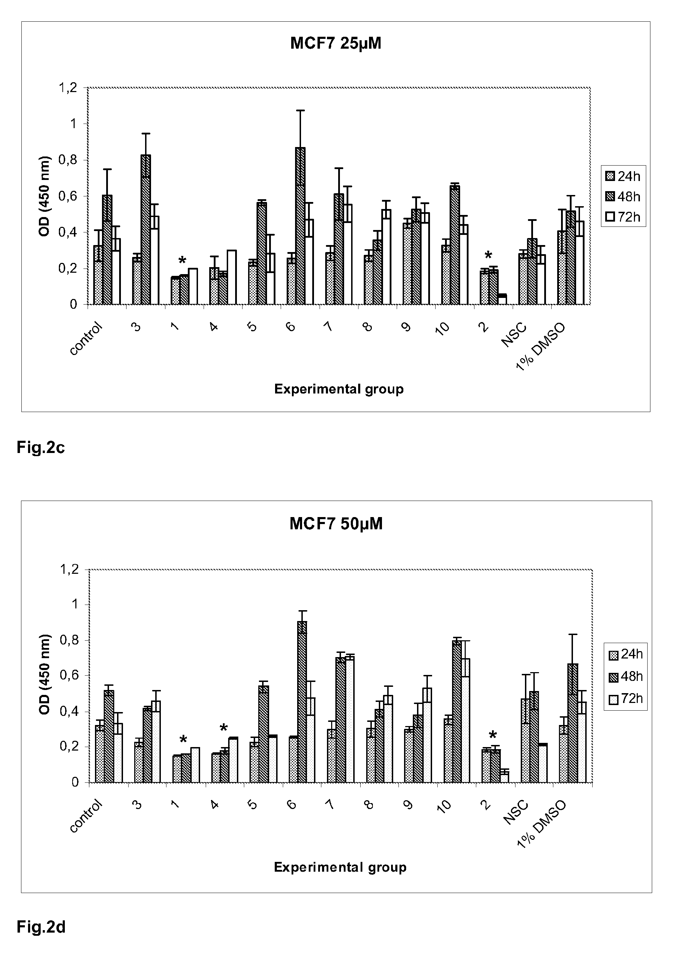

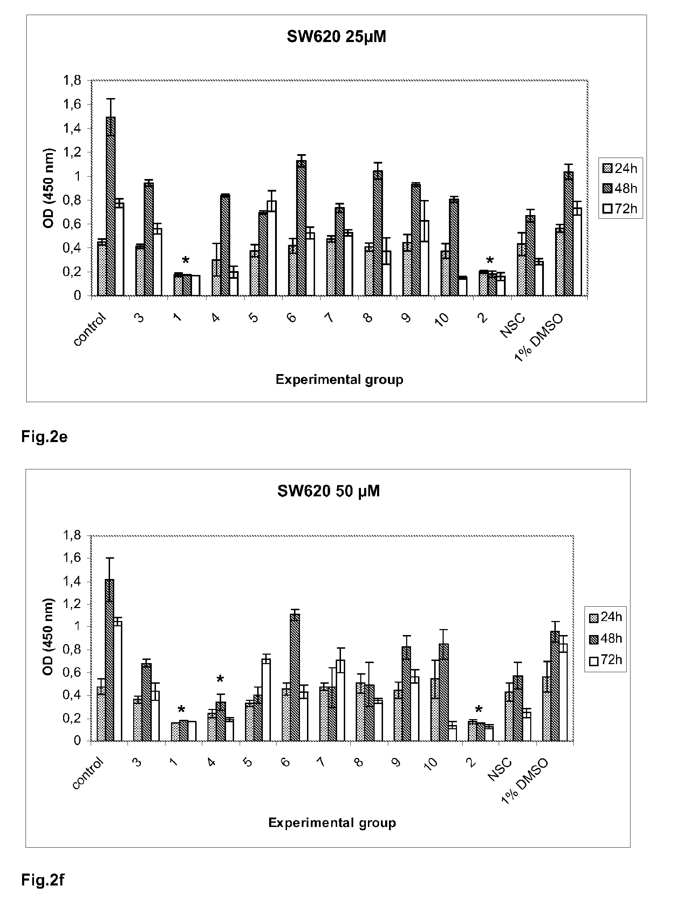

Rac-1 inhibitor compounds were tested in cell proliferation assays performed with following cancer cell lines: MCF7 (mammary carcinoma), SW620 (colon carcinoma), CRL 2505 (prostate carcinoma). The level of proliferation was measured at 24 hours (h), 48 h and 72 h after in vitro induction of compounds. Two different concentrations of compounds were tested for each cell line (25 and 50 µM, see Figs. 2a-f; the asteriks indicates significant change versus control group (no compound induction) at all incubation points (p<0.02)).

The proliferation assay was performed with Cell Proliferation Reagent WST-1 (Roche Cat. No. 1644 807). The cells were cultured 2 x 10⁴ cells/well in microplates (96 wells, flat bottom). The Cell Proliferation Reagent WST-1 (10 µl/well) was added to each experimental group (Figs. 2a-f). After 4 hours of incubation (37°C) the absorbance was measured by Microplate (ELISA) reader at 450 nm optical density (OD). The reference wavelength was 620 nm.

All experimental compounds (compounds 1, 2, 3-10, 18, 53) were solved in 1% DMSO solution. Therefore, additional experimental group (1% DMSO) was used to examine proliferation activity of used cell lines in presence of 1% DMSO.

All measurements were corrected to the blank absorbance (medium with 10 µl Cell Proliferation Reagent WST-1, no cells).

The results clearly show that compounds 1 and 2 are effective in inhibiting proliferation of the tumour cells tested. The effect of the compounds according to the present invention is significantly improved compared to the known Rac-1 inhibitor compound NSC23766 (Canselas et al., Nat. Med. 11 (2005), 886-891).

### Example 3

### MCF-7 Rac-1 Inhibition

Female athymic nude mice (Harlan Winkelmann), 6 week of age, were weighed, coded and randomly assigned to 4 experimental groups of n=8. This experiment was approved by the Institutional Animal Care and Use Committee at the Vienna Medical University.

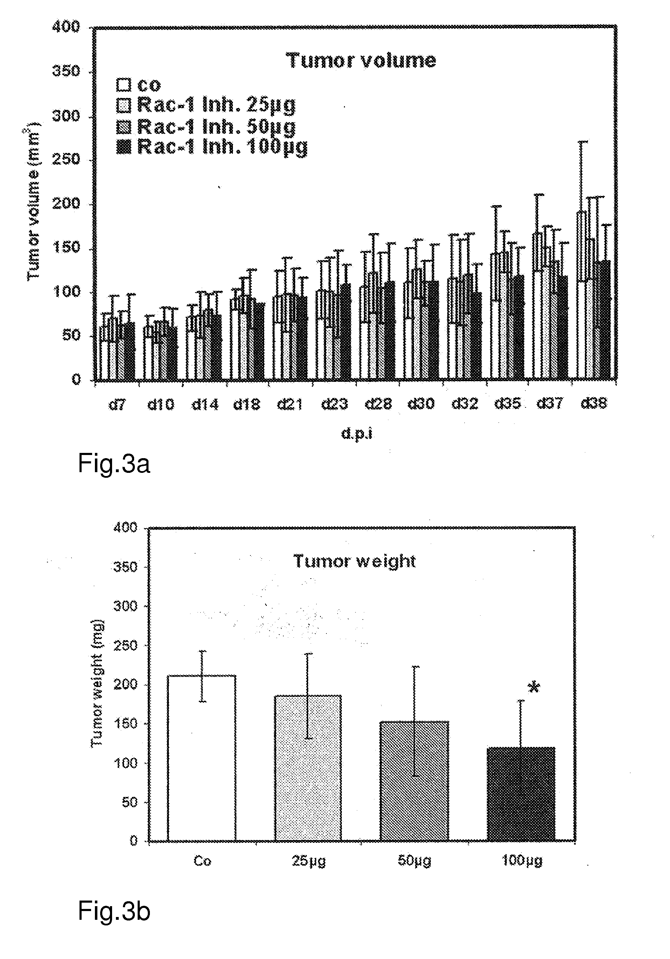

20 x 10⁷ MCF-7 cells/200µl PBS were injected subcutaneously into the left flank. All mice developed tumours of comparable size. On the indicated days, tumour sizes were determined with a caliper and tumour volumes calculated according to the following formula: tumour volume = [(length x width²) x n/6]. Figure 3a shows the tumour volumes of xenograft tumours measured on the indicated days (dpi = days post injection).

On day 24, mice were anaesthetized (ketamine hydrochloride/xylazine at 55/7.5 mg/kg, s.c.) and osmotic minipumps with different Rac-1 inhibitor (compound # 2) concentrations (25µg, 50µg and 100µg/day release) were implanted intraperitoneally. Control mice received osmotic pumps filled with Ringer's solution. All mice were sacrificed on day 38 and tumour weights were then determined. Fig. 3b shows the mean tumour weight on day 38 (mean breast cancer xenograft masses in control mice and mice treated with 25, 50 and 100µg of Rac-1 inhibitor; asterisk: significantly different from control (Co), P<0.05).

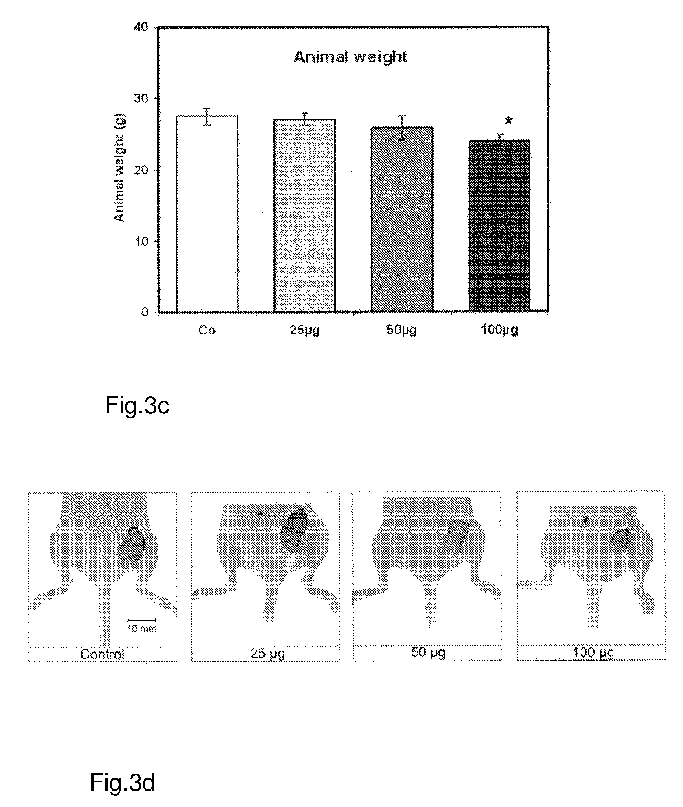

Fig. 3c shows body weights of mice bearing human breast cancer xenografts; asterisk: significantly different from control (Co), P<0.05).

Control mice developed tumours with a mean tumour weight of 210 ± 31 mg. Mice treated with 25µg, 50µg and 100µg Rac-1 inhibitor per day developed tumours with mean tumour weights of 185 ± 54mg, 152 ± 70mg and 118 ± 60mg, respectively. Fig. 3d shows representative images of mice bearing breast cancer xenografts treated with 25µg, 50µg and 100µg of Rac-1 inhibitor.

### Example 4

SW620-Colon Cancer Rac-1-Inhibitor Studies in Athymic Nude Mice 4 Compounds (#1, 2, 18, 53) with the following structure according to the residue nomenclature according to formula (I) were tested in a mouse xenograft model of human colon cancer:

**Table 1: compounds tested in vivo in the colon cancer model**

| | **R1** | **R2** | **R3** | **R4** | **R5** | **A** | **B** | **R6** | **R8** | **n** | **R9** | **R10** |
|---|---|---|---|---|---|---|---|---|---|---|---|---|
| 1 | | H | H | H | H | CH2CH2 | 6-Me-Pyr | H | CH3 | 3 | C2H5 | C2H5 |
| **2** | H | **OH** | H | H | H | CH2CH2 | 6-Me-Pyr | H | CH3 | 3 | C2H5 | C2H5 |
| **18** | H | OH | H | H | H | CH2CH2 | **Pyr** | H | CH3 | 3 | C2H5 | C2H5 |
| **53** | H | H | H | H | H | CH2CH2 | **5-Br-Pyr** | H | CH3 | 3 | C2H5 | C2H5 |

Experimental groups:
1) Ringer-Control (Co)
2) NSC23766 (NSC), known Rac-Inhibitor, comparative control
3) Compound # 1
4) Compound # 2
5) Compound # 18 comparative control
6) Compound # 53 comparative control

Male athymic nude mice (Harlan Winkelmann), 6 week of age, were weighed, coded and randomly assigned to 6 experimental groups (Control, known Rac-1 inhibitor compound NSC23766, Compounds #2, #18, #1, and #53). This experiment was approved by the Institutional Animal Care and Use Committee at the Vienna Medical University.

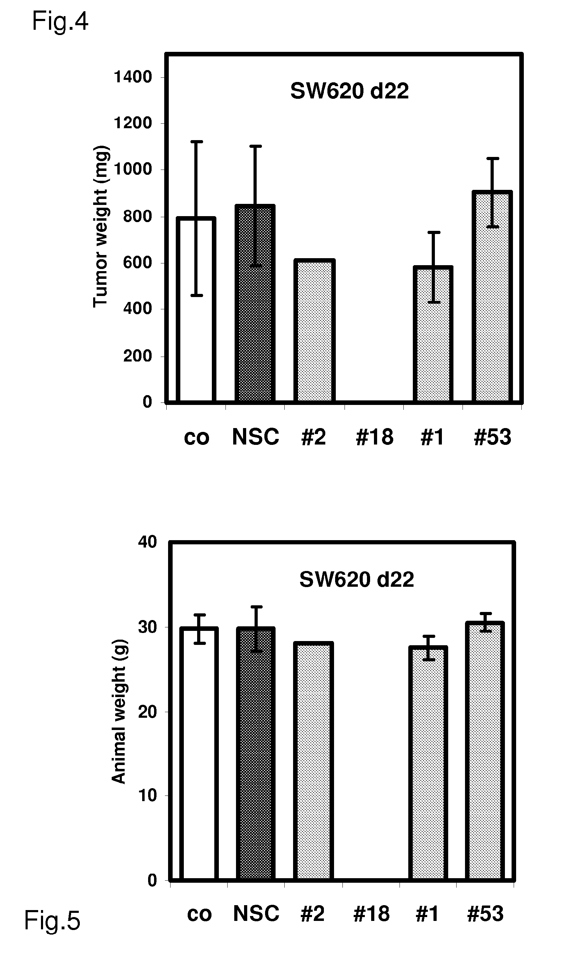

8 x 10⁶ SW620 cells/100µl PBS were injected subcutaneously into the left flank. All mice developed tumours of comparable size. On day 8 after tumour cell engrafment, mice were anesthetized (ketamine hydrochloride/xylazine at 55/7.5 mg/kg, s.c.) and osmotic minipumps with different Rac-1 inhibitors (known Rac-1 inhibitor compound NSC23766, Compounds #2, #18, #1, and #53) at a concentration of 100µg/day (100µg release per day for 2 weeks from osmotic minipump) were implanted intraperitoneally. Control mice received osmotic pumps filled with Ringer's solution. All mice were sacrificed on day 22 and tumour weights were then determined. The results are disclosed in Table 2 and Figs. 4 and 5.

Figure 4 shows the mean colon cancer xenograft masses in control mice and mice treated with 100µg/day of different Rac-1 inhibitors on day 22. Figure 5 shows body weights of mice bearing human colon cancer xenografts on day 22.

**Table 2: results of the in vivo tests in the SW620 colon cancer model**

| Mean tumour weight (±SD) | |
|---|---|
| Co: | 793 ± 328 mg |
| NSC: | 846 ± 256 mg |
| #2: | 612 |
| #18: | (Death of all animals following osmotic minipump implantation) |
| #1: | 583 ± 151 |
| #53: | 904 ± 146 |

| | Mean tumour weight (mg) | Mean tumour weight (% of control (co)) |
|---|---|---|
| Co | 793 | 100 |
| NSC | 846 | 107 |
| #2 | 612 | 77 |
| #18 | Death of all animals following osmotic minipump implantation) | |
| #1 | 583 | 74 |
| #53 | 904 | 114 |

## Claims

1. : Use of a 1H-indol-3-yl-compound with the formula (I) 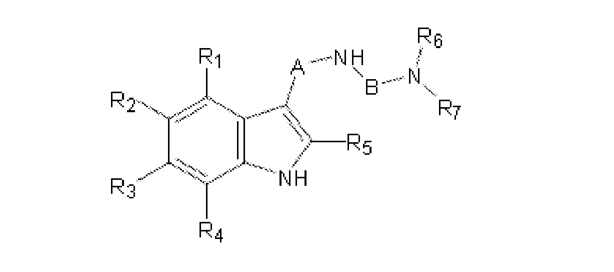 and any of its stereoisomeric forms or a mixture thereof
wherein
R₁, R₃, R₄ and R₅ are H;
R₂ is H or OH;
A is CH₂-CH₂;
B is 6-CH₃-pyrimidine;
R₆ is H; and
R₇ is CHR₈(CH₂)₃NR₉R₁₀; wherein R₈ is CH₃; R₉ and R₁₀ are C₂H₅;
or a salt or solvate of a compound of formula (I) for the treatment of malignant diseases.

2. : N2-(4-Diethylamino-1-methyl-butyl)-N4-[2-(1H-indol-3-yl)-ethyl]-6-methyl-pyrimidine-2,4-diamine and 3-{2-[2-(4-Diethylamino-1-methyl-butylamino)-6-methyl-pyrimidin-4-ylamino]-ethyl}-1H-indol-5-ol or a salt or solvate thereof for the treatment of malignant diseases.

3. : A pharmaceutical preparation containing a 1H-indol-3-yl-compound according to claim 1 or 2 and a pharmaceutically acceptable carrier.

4. : A pharmaceutical preparation containing a 1H-indol-3-yl-compound according to claim 1 or 2 and a pharmaceutically acceptable carrier for administration by injection.

5. : Use according to claim 1 **characterised in that** said malignant disease is a carcinoma, preferably breast, colon, rectum, esophagus, stomach, liver, lung, renal, thyroid, ovary, uterus, vagina, pancreas, bladder, prostate, skin, head and neck squamous cell carcinoma (HNSCC), and oral squamous cell carcinoma (OSCC), a hematopoietic malignancy of lymphoid lineage, preferably anaplastic large cell lymphomas (ALCLs), p210-BCR-ABL-dependent leukemia and hairy cell leukemia), a hematopoietic malignancy of myeloid lineage, preferably chronic myelogenous leukemias (CML), a malignancy of mesenchymal origin, preferably rhabdomyosarcoma, or a malignant tumour of neuronal origin, especially glioblastoma.

6. : Use according to claim 1 **characterised in that** said malignant disease is breast cancer or colon cancer.

7. : Use of a compound as defined in claim 1 or 2 for the preparation of a pharmaceutical composition for the treatment of a malignant disease.
